# EUROPÄISCHE PATENTSCHRIFT

(11) **EP 2 983 815 B1**
(45) Veröffentlichungstag und Bekanntmachung des Hinweises auf die Patenterteilung: **14.08.2019**
(21) Anmeldenummer: 14712266.7
(22) Anmeldetag: 20.03.2014
(51) Int. Cl.: C01G 51/00, C01G 53/00, C07C 45/35, B01J 23/00, B01J 23/887, B01J 37/00, B01J 37/02, B01J 35/02, B01J 35/10

(54) **KOMPOSITMATERIAL ENTHALTEND EIN BISMUT-MOLYBDÄN-NICKEL-MISCHOXID ODER EIN BISMUT-MOLYBDÄN-COBALT-MISCHOXID UND SIO2**
COMPOSITE MATERIAL CONTAINING A BISMUTH-MOLYBDENUM-NICKEL MIXED OXIDE OR A BISMUTH-MOLYBDENUM-COBALT MIXED OXIDE AND SIO2
MATÉRIAU COMPOSITE CONTENANT UN OXYDE MIXTE DE BISMUTH, MOLYBDÈNE ET NICKEL OU UN OXYDE MIXTE DE BISMUTH, MOLYBDÈNE ET COBALT ET DU SIO2

(30) Priorität: 20.03.2013 DE 102013004755
(43) Veröffentlichungstag der Anmeldung: 17.02.2016
(73) Patentinhaber: Clariant International Ltd, 4132 Muttenz (CH)
(72) Erfinder: WALZEL, Inga, 81369 München (DE); MESTL, Gerhard, 80935 München (DE); NEUMANN, Silvia, 83109 Großkarolinenfeld (DE); PRITZL, Magdalena, 83052 Bruckmühl (DE); DONABAUER, Gabriele, 83052 Bruckmühl (DE); GRASSELI, Robert K, 81247 München (DE)
(74) Vertreter: Kuba, Stefan
(86) Internationale Anmeldenummer: PCT/EP2014/055625
(87) Internationale Veröffentlichungsnummer: WO 2014/147187

(56) Entgegenhaltungen:
- EP-A1- 1 386 661
- EP-A1- 1 449 579
- WO-A1-2013/007736
- DE-A1-102005 037 678
- DE-A1-102008 017 308
- FR-A1- 2 502 977
- Evonik Industries: "Inorganic Materials for Catalyst Innovation", Industry Information 2242, 2011, Retrieved from the Internet: URL:http://www.sipernat.com/sites/dc/Downl oadcenter/Evonik/ Product/SIPERNAT/11-01-344-vnd-ii-2242-kat alysatoren-us.pdf [retrieved on 2014-06-03]

## Beschreibung

Die vorliegende Erfindung betrifft ein Verfahren zur Herstellung eines Kompositmaterials sowie das Kompositmaterial selbst. Das Kompositmaterial enthält ein Bismut-Molybdän-Nickel-Mischoxid oder ein Bismut-Molybdän-Cobalt-Mischoxid und ein spezielles SiO₂ als Porenbildner. Die vorliegende Erfindung betrifft auch die Verwendung des erfindungsgemäßen Kompositmaterials zur Herstellung einer Washcoat-Suspension sowie ein Verfahren zur Herstellung eines Schalenkatalysators unter Verwendung des erfindungsgemäßen Kompositmaterials. Weiterhin betrifft die vorliegende Erfindung auch einen Schalenkatalysator, der auf einem Trägerkörper eine katalytisch aktive Schale aufweist, die das erfindungsgemäße Kompositmaterial umfasst. Der erfindungsgemäße Schalenkatalysator wird zur Herstellung von α,β-ungesättigten Aldehyden aus Olefinen verwendet.

Molybdän-haltige Mischoxide werden bisher im Stand der Technik als Katalysatoren zur Herstellung von Acrylnitril aus Propen durch Ammoxidation oder zur Herstellung von α,β-ungesättigten Aldehyden aus Olefinen eingesetzt. Hierbei werden diese Molybdänmischoxide üblicherweise durch Fällungsmethoden, Sol-Gel-Verfahren oder Festkörperreaktionen erhalten. Bekannt im Stand der Technik ist auch, dass Molybdänmischoxide durch Einsprühen einer Lösung von Ausgangsmaterialien in einen Pulsationsreaktor mit einer kleinen Partikelgröße und einer möglichst großen BET-Oberfläche hergestellt werden können.

Obwohl insbesondere die Molybdänmischoxide mit kleiner Partikelgröße und möglichst großer BET-Oberfläche sich äußerst gut zur Herstellung von Katalysatoren eignen, so besteht weiterhin ein Verbesserungsbedarf der genannten Katalysatoren hinsichtlich ihrer Aktivität und Selektivität. Gleichzeitig besteht auch der Bedarf daran, die Menge der relativ teuren Ausgangssubstanzen bei der Herstellung solcher Katalysatoren zu minimieren und trotzdem einen Katalysator mit einer guten Performance bereitzustellen.

Das Dokument DE102005037678A1 offenbart ein Verfahren zur Herstellung von Katalysatorformkörpern.

Das Dokument DE102008017308A1 betrifft ein Verfahren zur Herstellung eines nanokristallinen Bismut-Molybdänmischoxidmaterials, und die Verwendung des Bismut-Molybdänmischoxidmaterials als Katalysator für chemische Umsetzungen.

Das Dokument WO2013/007736A1 beschreibt Mo, Bi und Fe enthaltende Multimetalloxidmassen.

Das Dokument FR2502977 betrifft einen Katalysator, der aktiv bei der Umwandlung von ungesättigten Kohlenwasserstoffen ist, sowie ein Verfahren zur Herstellung des Katalysators. Es war deshalb Aufgabe der vorliegenden Erfindung, ein Kompositmaterial als Aktivmasse für einen Katalysator bereitzustellen, der eine gute katalytische Performance aufweist. Zudem war es auch eine Aufgabe der vorliegenden Erfindung, ein Verfahren zur Herstellung eines Kompositmaterials bzw. eines Katalysator bereitzustellen, bei dem die Menge an verwendeten Aktivmetallen möglichst gering gehalten werden kann, ohne dass der Katalysator an katalytischer Performance im Vergleich mit Katalysatoren des Standes der Technik verliert.

Zur Lösung der genannten Aufgaben stellt die vorliegende Erfindung ein Verfahren zur Herstellung eines Kompositmaterials bereit, das die folgenden Schritte umfasst:
(a) Herstellen einer ersten wässrigen Lösung enthaltend Salze des Bismuts und Nickels oder Salze des Bismuts und Cobalts, vorzugsweise Salze des Bismuts und Nickels, sowie zusätzlich ein Salz des Magnesiums ;
(b) Herstellen einer zweiten wässrigen Lösung enthaltend eine Molybdänverbindung und gegebenenfalls ein Bindemittel;
(c) Zugabe der ersten wässrigen Lösung zu der zweiten wässrigen Lösung, wobei eine erste Suspension gebildet wird;
(d) Zugabe einer zweiten Suspension zu der ersten Suspension, wobei die zweite Suspension SiO₂ enthält, das ein Porenvolumen im Bereich von 0,1 bis 10 ml/g und eine durchschnittliche Teilchengröße im Bereich von 3 bis 20 µm aufweist; und
(e) Sprühkalzinieren der dritten Suspension bei einer Temperatur im Bereich von 300 bis 600°C unter Erhalt des Kompositmaterials enthaltend ein Bismut-Molybdän-Nickel-Mischoxid oder ein Bismut-Molybdän-Cobalt-Mischoxid, vorzugsweise ein Bismut-Molybdän-Nickel-Mischoxid.

Die Begriffe "Bismut-Molybdän-Cobalt-Mischoxid" und "Bismut-Molybdän-Nickel-Mischoxid" sollen in der vorliegenden Anmeldung als ein Mischoxid verstanden werden, das Bismut, Molybdän und Cobalt bzw. Bismut, Molybdän und Nickel als oxidische Metalle umfasst.

In einer weiterhin bevorzugten Ausführungsform kann die erste wässrige Lösung in Schritt (a) zusätzlich ein Salz des Eisens enthalten.

In einer noch weiterhin bevorzugten Ausführungsform kann die erste wässrige Lösung eine Kaliumverbindung enthalten. In einer noch weiteren Ausführungsform kann die erste wässrige Lösung sowohl eine Kalium- als auch eine Cäsiumverbindung oder eine Kalium- und Cäsium-haltige Verbindung enthalten.

Die erste wässrige Lösung wird vorzugsweise dadurch hergestellt, dass die darin eingesetzten Metallsalze bei einer Temperatur im Bereich von 40 bis 60°C vorzugsweise unter Rühren in einer möglichst geringen Menge entionisiertem Wasser gelöst werden.

In einer weiterhin bevorzugten Ausführungsform wird die erste wässrige Lösung so hergestellt, dass zunächst Wasser unter Rühren bei einer Temperatur im Bereich von 40 bis 60°C vorgelegt und zunächst das Nickelsalz und anschließend das Bismutsalz hinzugegeben wird. Soll die erste wässrige Lösung eine Kaliumverbindung enthalten, so ist die Reihenfolge der Zugabe vorzugsweise folgendermaßen: Zugabe des Nickelsalzes, Zugabe der Kaliumverbindung und anschließend die Zugabe des Bismutsalzes. Wird bei der Herstellung der ersten wässrigen Lösung auch ein Eisensalz eingesetzt, so erfolgt die Zugabe des Eisensalzes vorzugsweise vor der Zugabe des Nickelsalzes.

Die Zugabe des Magnesiumsalzes erfolgt vorzugsweise nach der Zugabe des Nickelsalzes aber vor der Zugabe der Kaliumverbindung bzw. vor der Zugabe der Bismutverbindung.

Es ist besonders bevorzugt, dass als Eisen-, Nickel- bzw. Magnesiumsalze wasserlösliche Salze dieser Metalle eingesetzt werden. Insbesondere bevorzugt werden die Nitrate dieser Metallsalze eingesetzt. In einer ganz besonders bevorzugten Ausführungsform der vorliegenden Erfindung werden zur Herstellung der ersten wässrigen Lösung Metallnitrat-Hydrate eingesetzt. Als Eisensalz wird vorzugsweise Eisennitrat-Nonahydrat, als Nickelsalz vorzugsweise Nickelnitrat-Hexahydrat und als Magnesiumsalz vorzugsweise Magnesiumnitrat-Hexahydrat eingesetzt. Alternativ oder zusätzlich zu dem Einsatz eines Nickelsalzes kann zur Herstellung der ersten wässrigen Lösung auch ein Cobaltsalz, vorzugsweise ein wasserlösliches Cobaltsalz wie Cobaltnitrat-Hexahydrat eingesetzt werden. Das Cobaltsalz wird - was die Reihenfolge der Zugabe der Komponenten betrifft - an der gleichen Stelle wie das Nickelsalz zugegeben.

Das Bismutsalz ist vorzugsweise eines, das sich beim Erhitzen bis auf 60°C verflüssigt. Der Einsatz eines Bismutsalzes, das bei einer Temperatur zwischen 40 und 60°C schmilzt, liegt darin, dass Bismutverbindungen in der Regel im Wasser schwer lösliche Verbindungen sind, die nur durch starkes Ansäuern in Lösung gebracht werden können. Es ist erfindungsgemäß deshalb insbesondere bevorzugt, ein Hydratsalz des Bismuts zur Herstellung der ersten wässrigen Lösung einzusetzen, das bei Temperaturen zwischen 40 und 60°C durch seinen hohen Hydratwassergehalt in Form einer sogenannten wässrigen Schmelze vorliegt. Deshalb ist es auch in der vorliegenden Erfindung bevorzugt, dass sämtliche zur Herstellung der ersten wässrigen Lösung eingesetzten Metallsalze Hydrat-Salze dieser Verbindungen sind. Auf diese Weise muss zur Herstellung der ersten wässrigen Lösung nicht unbedingt Wasser zum Lösen vorgelegt werden, sondern es können die eingesetzten Hydratmetallsalze nacheinander unter Rühren bei einer Temperatur zwischen 40 und 60°C zu einer wässrigen Schmelze verflüssigt werden. Auf diese Weise kann sämtliches eingesetztes Bismutsalz auch weiterverarbeitet werden. Gegebenenfalls kann zur vollständigen Lösung auch - vorzugsweise mit Salpetersäure - angesäuert werden. Im Stand der Technik bei der Herstellung von Bismut-haltigen Lösungen musste immer eine überstöchiometrische Menge Bismut eingesetzt werden, um die gewünschte Menge an Bismut vorliegen zu haben.

Als Kaliumverbindung können erfindungsgemäß alle wasserlöslichen Kaliumverbindungen bzw. Kaliumsalze eingesetzt werden. Es ist erfindungsgemäß jedoch bevorzugt, dass als Kaliumverbindung Kaliumnitrat oder Kaliumhydroxid, stärker bevorzugt Kaliumhydroxid eingesetzt wird.

Die Menge des Bismutsalzes in der ersten Lösung liegt vorzugsweise im Bereich von 3 bis 9 Gew.-%, stärker bevorzugt im Bereich von 4 bis 8 Gew.-% und am stärksten bevorzugt im Bereich von 5 bis 7 Gew.-%, bezogen auf das Gesamtgewicht der ersten Lösung inklusive Wasser.

Die Menge des Nickelsalzes oder des Cobaltsalzes oder deren Gesamtmenge in der ersten Lösung liegt vorzugsweise im Bereich von 35 bis 60 Gew.-%, stärker bevorzugt im Bereich von 40 bis 55 Gew.-% und am stärksten bevorzugt im Bereich von 45 bis 50 Gew.-%, bezogen auf das Gesamtgewicht der ersten Lösung inklusive Wasser.

Die Menge des Eisensalzes in der ersten Lösung liegt vorzugsweise im Bereich von 0 bis 40 Gew.-%, stärker bevorzugt im Bereich von 1 bis 35 Gew.-%, noch stärker bevorzugt im Bereich von 5 bis 30 Gew.-% und am stärksten bevorzugt im Bereich von 15 bis 25 Gew.-%, bezogen auf das Gesamtgewicht der ersten Lösung inklusive Wasser. Enthält die erste Lösung ein Eisensalz, so bildet sich im erfindungsgemäßen Verfahren ein Mischoxid, das auch Eisen in Form von Eisenoxid enthält. Die Menge des Magnesiumsalzes in der ersten Lösung liegt vorzugsweise im Bereich oberhalb von 0 bis 25 Gew.-%, stärker bevorzugt im Bereich von 1 bis 20 Gew.-%, noch stärker bevorzugt im Bereich von 5 bis 17 Gew.-% und am stärksten bevorzugt im Bereich von 10 bis 14 Gew.-%, bezogen auf das Gesamtgewicht der ersten Lösung inklusive Wasser. Enthält die erste Lösung ein Magnesiumsalz, so bildet sich im erfindungsgemäßen Verfahren ein Mischoxid, das auch Magnesium in Form von Magnesiumoxid enthält.

Die Menge der Kaliumverbindung in der ersten Lösung liegt vorzugsweise im Bereich von 0 bis 0,1 Gew.-%, stärker bevorzugt im Bereich von 0,005 bis 0,08 Gew.-%, noch stärker bevorzugt im Bereich von 0,02 bis 0,06 und am stärksten bevorzugt im Bereich von 0,03 bis 0,05 Gew.-%, bezogen auf das Gesamtgewicht der ersten Lösung inklusive Wasser.

In Schritt (b) des erfindungsgemäßen Verfahrens wird die zweite wässrige Lösung enthaltend eine Molybdänverbindung und gegebenenfalls ein Bindemittel dadurch hergestellt, dass die vorzugsweise wasserlösliche Molybdänverbindung vorgelegt und anschließend in Wasser gelöst wird. Wird zusätzlich ein Bindemittel eingesetzt, so wird zunächst die Molybdänverbindung und dann das Bindemittel vorgelegt und anschließend in Wasser gelöst.

Als Molybdänverbindung kann jede denkbare wasserlösliche Molybdänverbindung eingesetzt werden, es ist jedoch bevorzugt, dass die Molybdänverbindung ein Molybdat ist. Besonders bevorzugt ist die Molybdänverbindung ein Heptamolybdat, stärker bevorzugt ein Ammoniumheptamolybdat und am stärksten bevorzugt Ammoniumheptamolybdat-Tetrahydrat.

Das optional zur Herstellung der zweiten wässrigen Lösung eingesetzte Bindemittel ist vorzugsweise ein SiO₂-haltiges Bindemittel. Dieses Bindemittel weist vorzugsweise einen Gehalt an Natrium von kleiner als 400 ppm, stärker bevorzugt kleiner als 300 ppm und am stärksten bevorzugt kleiner als 250 ppm auf. Ist das Bindemittel ein SiO₂-haltiges Bindemittel, so weist das SiO₂ vorzugsweise eine BET-Oberfläche von im Bereich von 140 bis 220 m²/g, stärker bevorzugt 160 bis 200 m²/g und am stärksten bevorzugt 180 bis 190 m²/g auf. Die Dichte des SiO₂ liegt hierbei vorzugsweise im Bereich von 1,00 bis 1,40 g/cm³, stärker bevorzugt 1,15 bis 1,30 g/cm³ und am stärksten bevorzugt im Bereich von 1,20 bis 1,25 g/cm³.

Die Menge der Molybdänverbindung in der zweiten Lösung liegt vorzugsweise im Bereich von 5 bis 40 Gew.-%, stärker bevorzugt im Bereich von 10 bis 35 Gew.-% und am stärksten bevorzugt im Bereich von 18 bis 26 Gew.-%, bezogen auf das Gesamtgewicht der zweiten Lösung inklusive Wasser.

Die Menge des Bindemittels in der zweiten Lösung liegt vorzugsweise im Bereich von 0 bis 20 Gew.-%, stärker bevorzugt im Bereich von 1 bis 17 Gew.-%, noch stärker bevorzugt im Bereich von 4 bis 13 und am stärksten bevorzugt im Bereich von 6 bis 11 Gew.-%, bezogen auf das Gesamtgewicht der zweiten Lösung inklusive Wasser.

Es ist weiterhin bevorzugt, dass die Zugabe der ersten wässrigen Lösung zu der zweiten wässrigen Lösung in Schritt (c) des erfindungsgemäßen Verfahrens so durchgeführt wird, dass die zweite wässrige Lösung mit Hilfe eines Rührwerks schnell gerührt wird, und die erste wässrige Lösung langsam zu der zweiten wässrigen Lösung zugetropft oder zugepumpt wird. Dabei bildet sich ein Niederschlag, so dass dadurch in Schritt (c) eine erste Suspension gebildet wird.

Weiterhin wird eine zweite Suspension getrennt von der ersten gebildeten Suspension hergestellt, die ein SiO₂ in Wasser enthält, das ein Porenvolumen im Bereich von 0,1 bis 10 ml/g, stärker bevorzugt 0,5 bis 5 ml/g und am stärksten bevorzugt 1 bis 3 ml/g und eine durchschnittliche Teilchengröße im Bereich von 3 bis 20 µm, stärker bevorzugt 5 bis 15 µm und am stärksten bevorzugt 8 bis 11 µm aufweist. Weiterhin bevorzugt ist es, dass das zur Herstellung der zweiten Suspension eingesetzte SiO₂ eine Ölabsorption im Bereich von 250 g/100 g bis 400 g/100 g, stärker bevorzugt 300 g/100 g bis 350 g/100 g aufweist.

Die Menge an SiO₂ in der zweiten Suspension liegt im Bereich von 1 bis 20 Gew.-%, stärker bevorzugt im Bereich von 2 bis 10 Gew.-% und am stärksten bevorzugt im Bereich von 3 bis 7 Gew.-%, bezogen auf das Gesamtgewicht der Suspension inklusive Wasser.

In Schritt (d) des erfindungsgemäßen Verfahrens wird die zweite Suspension vorzugsweise unter Rühren der ersten Suspension zu dieser gegeben.

Die Menge an zweiter Suspension wird in dem erfindungsgemäßen Verfahren vorzugsweise so gewählt, dass das Gewichtsverhältnis der eingesetzten Molybdänverbindung in der ersten Suspension zu dem SiO₂ der zweiten Suspension im Bereich von 5 bis 25, stärker bevorzugt im Bereich von 10 bis 20 und am stärksten bevorzugt im Bereich von 12 bis 16 liegt.

Ebenso liegt das Gewichtsverhältnis des eingesetzten Nickel- oder Cobaltsalzes in der ersten Suspension zu dem SiO₂ in der zweiten Suspension im Bereich von 5 bis 25, stärker bevorzugt im Bereich von 10 bis 20 und am stärksten bevorzugt im Bereich von 11 bis 15.

Zusätzlich zu der Zugabe der zweiten Suspension zu der ersten Suspension kann in Schritt (d) noch ein Fällungsmittel zu der ersten Suspension hinzugegeben werden. Das Fällungsmittel ist vorzugsweise ein kationisches Polymer, wobei ein kationisches Polyacrylamidderivat bevorzugt ist. Die Menge des zuzugebenden Fällungsmittels wird vorzugsweise so gewählt, dass das Gewichtsverhältnis von Molybdänverbindung zu Fällungsmittel im Bereich von 1,3 x 10² bis 1,3 x 10⁴, stärker bevorzugt im Bereich von 0,3 x 10³ bis 0,3 x 10⁴ und am stärksten bevorzugt im Bereich von 1,1 x 10³ bis 1,7 x 10³ liegt.

Aus der Vereinigung der ersten und zweiten Suspension in Schritt (d) des erfindungsgemäßen Verfahrens entsteht eine dritte Suspension, die im anschließenden Schritt (e) des erfindungsgemäßen Verfahrens bei einer Temperatur im Bereich von 300 bis 600°C, stärker bevorzugt 300 bis 550°C und stärker bevorzugt 450 bis 520°C sprühkalziniert wird. Dabei entsteht ein Kompositmaterial, das ein Bismut-Molybdän-Nickel-Mischoxid oder ein Bismut-Molybdän-Cobalt-Mischoxid enthält.

Unter dem Begriff "Sprühkalzinieren" versteht man erfindungsgemäß, dass die Suspension in einen Kalzinierreaktor bzw. Kalzinierofen, der im angegebenen Temperaturbereich vorgeheizt ist, eingesprüht wird. Auf diese Weise verdampft die Flüssigkeit und es entsteht das Kompositmaterial. Es ist besonders bevorzugt, dass als Kalizinieröfen bzw. Kalzinierreaktoren solche eingesetzt werden, wie sie beispielsweise in der Anmeldung mit der Veröffentlichungsnummer DE 10 2008 017 311 zur Herstellung von Mischoxiden eingesetzt werden.

Nach dem Sprühkalzinieren kann optional auch noch eine Kalzinierung in einem Kalzinierofen bei einer Temperatur im Bereich von 250 bis 450°C, stärker bevorzugt im Bereich von 300 bis 400°C durchgeführt werden.

Die vorliegende Offenbarung betrifft auch ein Kompositmaterial, das ein Bismut-Molybdän-Nickel-Mischoxid oder ein Bismut-Molybdän-Cobalt-Mischoxid und SiO₂ enthält, wobei das SiO₂ ein Porenvolumen im Bereich von 0,1 bis 10 ml/g und eine durchschnittliche Teilchengröße im Bereich von 3 bis 20 µm aufweist. Die weiter oben in Verbindung mit dem erfindungsgemäßen Verfahren in Schritt (d) angegebenen bevorzugten Parameterbereiche des in der zweiten Suspension eingesetzten SiO₂ sind auch für das im Kompositmaterial enthaltenen SiO₂ bevorzugt. In anderen Worten ist das in dem Kompositmaterial enthaltende SiO₂ das SiO₂, das im erfindungsgemäßen Verfahren in der zweiten Suspension eingesetzt wurde.

Demgemäß betrifft die vorliegende Erfindung auch ein Kompositmaterial, das nach dem erfindungsgemäßen Verfahren hergestellt wurde.

Durch den Einsatz des SiO₂ im erfindungsgemäßen Verfahren in Schritt (d) bzw. das Vorhandensein des SiO₂ mit dem angegebenen Porenvolumen und der angegebenen durchschnittlichen Teilchengröße hat den Vorteil, dass das Kompositmaterial ein bestimmtes Porenvolumen aufweist, das für den daraus hergestellten Katalysator wichtig für dessen hohe Aktivität und Selektivität ist.

Die BET-Oberfläche des erfindungsgemäßen Kompositmaterials liegt vorzugsweise im Bereich von 20 bis 60 m²/g, stärker bevorzugt im Bereich von 30 bis 50 m²/g und am stärksten bevorzugt im Bereich von 35 bis 45 m²/g.

Das Porenvolumen des erfindungsgemäßen Komposimaterials liegt vorzugsweise im Bereich von 0,08 bis 0,24 cm³/g und noch stärker bevorzugt im Bereich von 0,12 bis 0,20 cm³/g.

90% aller Teilchen des erfindungsgemäßen Kompositmaterials (d₉₀) weisen vorzugsweise eine Korngröße von kleiner als 125 µm auf. 50% aller Teilchen des erfindungsgemäßen Kompositmaterials (d₅₀) weisen vorzugsweise eine Korngröße von kleiner als 50 µm auf, wohingegen 10% aller Teilchen (d₁₀) vorzugsweise eine Korngröße von kleiner als 7 µm aufweisen.

Die vorliegende Erfindung betrifft auch die Verwendung des erfindungsgemäßen Kompositmaterials zur Herstellung einer Washcoat-Suspension.

In anderen Worten betrifft die vorliegende Erfindung auch eine Washcoat-Suspension, die das erfindungsgemäße Kompositmaterial enthält.

Zur Herstellung der erfindungsgemäßen Washcoat-Suspension wird erfindungsgemäßes Kompositmaterial in entionisiertem Wasser aufgeschlämmt. Anschließend wird die so hergestellte Washcoat-Suspension vorzugsweise einer Ultraturrax-Behandlung unterzogen, um zu gewährleisten, dass die Partikelgröße der Teilchen des Kompositmaterials im Bereich von 1 bis 100 µm liegt. Anschließend kann je nach Bedarf ein anorganischer und/oder organischer Porenbildner, ein Bindemittel und ein Klebstoff hinzugegeben werden. Werden diese weiteren Komponenten eingesetzt, so kann alternativ dazu auch die Vermahlung mit dem Ultraturrax nach der Zugabe dieser Komponenten erfolgen.

Die Menge des Kompositmaterials in der erfindungsgemäßen Washcoat-Suspension liegt vorzugsweise im Bereich von 1 bis 50 Gew.-%, stärker bevorzugt im Bereich von 5 bis 40 Gew.-% und am stärksten bevorzugt im Bereich von 10 bis 35 Gew.-%, bezogen auf das Gesamtgewicht der Washcoat-Suspension.

Als Bindemittel zur Herstellung der Washcoat-Suspension kann das gleiche Bindemittel eingesetzt werden, wie in dem Herstellungsverfahren des Kompositmaterials. Die Menge des Bindemittels in der erfindungsgemäßen Washcoat-Suspension liegt vorzugsweise im Bereich von 1 bis 15 Gew.-%, stärker bevorzugt im Bereich von 3 bis 12 Gew.-% und am stärksten bevorzugt im Bereich von 5 bis 10 Gew.-%, bezogen auf das Gesamtgewicht der Washcoat-Suspension.

Als anorganischer Porenbildner wird vorzugsweise ein SiO₂ eingesetzt, das die gleichen Eigenschaften wie das SiO₂ aufweist, das in der zweiten wässrigen Suspension in Schritt (d) des erfindungsgemäßen Verfahrens zur Herstellung des Kompositmaterials eingesetzt wird. Die Menge des anorganischen Porenbildners in der erfindungsgemäßen Washcoat-Suspension liegt vorzugsweise im Bereich von 0,1 bis 3 Gew.-%, stärker bevorzugt im Bereich von 0,5 bis 2 Gew.-% und am stärksten bevorzugt im Bereich von 0,6 bis 1 Gew.-%, bezogen auf das Gesamtgewicht der Washcoat-Suspension.

Als organischer Porenbildner kann jedweder organische Porenbildner mit einem hohen Kohlenstoffgehalt eingesetzt werden. So kann beispielsweise als organischer Porenbildner Kokosnussschalenmehl verwendet werden. Die Menge des organischen Porenbildners in der erfindungsgemäßen Washcoat-Suspension liegt vorzugsweise im Bereich von 0,5 bis 10 Gew.-%, stärker bevorzugt im Bereich von 1 bis 5 Gew.-% und am stärksten bevorzugt im Bereich von 2 bis 3 Gew.-%, bezogen auf das Gesamtgewicht der Washcoat-Suspension.

Als Klebstoff werden vorzugsweise anorganische Haftvermittler, wie Silane eingesetzt. Die Silane sind vorzugsweise Alkylalkoxysilane, wie zum Beispiel N-(2-aminoethyl)-3-aminopropylmethyldimethoxysilan. Die Menge des Klebstoffs in der erfindungsgemäßen Washcoat-Suspension liegt vorzugsweise im Bereich von 0,1 bis 3 Gew.-%, stärker bevorzugt im Bereich von 0,2 bis 2 Gew.-% und am stärksten bevorzugt im Bereich von 0,3 bis 1 Gew.-%, bezogen auf das Gesamtgewicht der Washcoat-Suspension.

Die Ultraturrax-Behandlung erfolgt vorzugsweise im Bereich von 1 bis 5 Minuten bei vorzugsweise 6000 bis 12000 U/min. Optional kann in der erfindungsgemäßen Washcoat-Suspension auch eine Alkaliverbindung, insbesondere Kalium- oder Cäsiumverbindung, wie Kaliumhydroxid, Cäsiumhydroxid, Kaliumnitrat oder Cäsiumnitrat eingesetzt werden. Die Gesamtmenge des/der Alkalimetalls/Alkalimetalle in der erfindungsgemäßen Washcoat-Suspension liegt hierbei vorzugsweise im Bereich von 0,05 bis 0,1, bezogen auf das Gesamtgewicht der Washcoat-Suspension. Weiterhin bevorzugt ist es, dass als Alkalimetall sowohl Kalium als auch Cäsium eingesetzt wird. Dabei ist es bevorzugt, dass das Atomverhältnis von Kalium zu Cäsium im Bereich von 1 bis 10, stärker bevorzugt im Bereich von 2 bis 8 und am bevorzugtesten im Bereich von 3 bis 6 liegt.

Die vorliegende Erfindung betrifft auch ein Verfahren zur Herstellung eines Schalenkatalysators, der die folgenden Schritte umfasst:
(a) Herstellen einer wässrigen Suspension des Kompositmaterials gemäß der Herstellung der erfindungsgemäßen Washcoat-Suspension;
(b) Beschichten von Trägerkörpern mit der in Schritt (a) hergestellten wässrigen Suspension; und
(c) Kalzinieren der beschichteten Trägerkörper bei einer Temperatur im Bereich von 500 bis 700°C, stärker bevorzugt 550 bis 650°C, noch stärker bevorzugt 590 bis 620°C und am stärksten bevorzugt etwa 610°C.

Das Kalzinieren in Schritt (c) des erfindungsgemäßen Verfahrens wird vorzugsweise über eine Zeitdauer im Bereich von 1 bis 5 h, stärker bevorzugt 2 bis 4 h und am stärksten bevorzugt etwa 3 h durchgeführt.

Es ist erfindungsgemäß bevorzugt, dass das Beschichten des Trägerkörpers so durchgeführt wird, dass auf eine einer Umwälzbewegung unterzogenen Schüttung von Trägerkörpern die in Schritt (a) hergestellte wässrige Suspension aufgesprüht wird.

Unter dem Begriff "Schalenkatalysator" versteht man einen Katalysator, der einen Trägerkörper und eine Schale mit katalytisch-aktivem Material umfasst. Dabei ist auf die Oberfläche des Trägerkörpers eine zusätzliche Schicht aufgebracht, in der ein katalytisch-aktives Material vorliegt. Diese Schicht bildet somit eine zusätzliche Materiallage, die als Schale um den Trägerkörper aufgebaut wird, d.h. das Trägermaterial selbst bildet keinen Bestandteil der Schale, sondern die Schale wird durch das katalytisch-aktive Material selbst oder einem Matrixmaterial, das ein katalytisch-aktives Material umfasst, gebildet.

Der Trägerkörper ist vorzugsweise aus einem inerten Material. Er kann porös oder nicht porös sein. Vorzugsweise ist der Trägerkörper jedoch nichtporös. Der Trägerkörper besteht vorzugsweise aus Teilchen mit einer regulären oder irregulären Form, wie beispielsweise Kugeln, Tabletten, Zylindern, Vollzylindern oder Hohlzylindern, Ringen, Sternen oder anderen Formen, und weist in seinen Dimensionen, wie zum Beispiel Durchmesser, Länge oder Breite einen Bereich von 1 bis 10 mm, bevorzugt 3 bis 9 mm auf. Sphärische, d.h. zum Beispiel kugelförmige Teilchen mit einem Durchmesser von 3 bis 8 mm sind erfindungsgemäß bevorzugt. Das Trägerkörpermaterial kann jegliche nicht poröse und poröse Substanz, vorzugsweise nichtporöse Substanz umfassen. Beispiele für Materialien hierfür sind Titanoxid, Siliziumoxid, Aluminiumoxid, Zirkoniumoxid, Magnesiumoxid, Siliziumcarbid, Magnesiumsilikat, Zinkoxid, Zeolithe, Schichtsilikate und Nanomaterialien, wie beispielsweise Kohlenstoffnanotubes oder Kohlenstoffnanofasern, Keramiken wie z.B. Steatit. Bevorzugtes Trägermaterial ist Steatit, besonders bevorzugt Steatitkugeln.

Es ist besonders bevorzugt, dass die Trägerkörper während des Aufbringens bzw. Aufsprühens der Suspension enthaltend das Kompositmaterial einer Umwälzbewegung unterzogen werden, so dass der Trägerkörper von allen Seiten gleichmäßig besprüht werden kann. Die Umwälzbewegung kann prinzipiell durch jegliches bekannte mechanische Rührgerät, wie beispielsweise eine Dragiertrommel erfolgen. Es ist jedoch erfindungsgemäß bevorzugt, dass die Umwälzbewegung der Trägerkörper mit Hilfe eines Prozessgases durchgeführt wird, z.B. in einem Fließbett, einer Wirbelschicht oder in einer Beschichtungskammer eines Innojet-Aircoaters. Dabei werden die Trägerkörper bewegt, indem das Prozessgas eingeblasen wird. Das Prozessgas wird hier vorzugsweise so geführt, dass die Trägerkörper in einer kontrollierten Gleitschicht des Prozesses gehalten werden. Das Prozessgas wird hier vorzugsweise erwärmt, so dass das Lösungsmittel rasch verdampft. Auf diese Weise liegen die Vorläuferverbindungen in der genannten definierten Schale des Trägerkörpers vor. Die Sprührate wird während des Aufsprühens vorzugsweise so gewählt, dass ein Gleichgewicht zwischen der Abdampfrate des Lösungsmittels und der Zuführungsrate der Vorläuferverbindungen auf dem Trägerkörper erreicht wird. Dies ermöglicht, die gewünschte Schalendicke in der Schale einzustellen. Je nach Sprührate kann somit die Schalendicke stufenlos eingestellt und optimiert werden, beispielsweise bis zu einer Dicke von 2 mm. Aber auch sehr dünne Schalen mit einer Dicke von kleiner als 200 µm sind so möglich.

Es ist besonders bevorzugt, dass die Sprührate bei dem Aufsprühen der Washcoat-Suspension konstant ist und im Bereich eines Massenstroms der Suspension von 0,5 bis 6 g/min pro 100 g zu beschichtendem Trägerformkörper, stärker bevorzugt im Bereich von 1 bis 4 g/min pro 100 g zu beschichtendem Trägerkörper, noch stärker bevorzugt 1 bis 3 g/min pro 100 g zu beschichtendem Trägerkörper liegt. In anderen Worten liegt das Verhältnis des Gewichts der aufgesprühten Suspension zu dem Gewicht der Schüttung des Trägerkörpers im Bereich von 0,005 bis 0,06, stärker bevorzugt 0,01 bis 0,04 und noch stärker bevorzugt 0,01 bis 0,03 Ein Massenstromverhältnis oberhalb des angegebenen Bereichs führt zu erheblichen Sprühverlusten bei der Katalysatorherstellung und somit zu erheblichem finanziellen Verlust, ein Massenstrom oder Verhältnis unterhalb des angegebenen Bereichs führt jedoch zu einem sehr hohen Zeitaufwand bei der Katalysatorherstellung und macht die Herstellung damit ineffizient.

Wird eine Fließbett-Anlage verwendet, ist es bevorzugt, wenn die Trägerkörper in dem Fließbett elliptisch oder torroidal umlaufen. Um eine Vorstellung davon zu geben, wie sich die Trägerkörper in derartigen Fließbetten bewegen, sei ausgeführt, dass bei "elliptischem Umlauf" sich die Trägerkörper in dem Fließbett in vertikaler Ebene auf einer elliptischen Bahn bewegen mit wechselnder Größe der Haupt- und Nebenachse. Bei "torroidalem Umlauf" bewegen sich die Trägerkörper in dem Fließbett in vertikaler Ebene auf einer elliptischen Bahn mit wechselnder Größe der Haupt- und Nebenachse und in horizontaler Ebene auf einer Kreisbahn mit wechselnder Größe des Radius. Im Mittel bewegen sich die Trägerkörper bei "elliptischem Umlauf" in vertikaler Ebene auf einer elliptischen Bahn, bei "torroidalem Umlauf" auf einer torroidalen Bahn, d.h., dass ein Trägerkörper die Oberfläche einen Torus mit vertikal elliptischem Schnitt helikal abfährt.

Weiterhin wird der in dem erfindungsgemäßen Verfahren zur Herstellung des Schalenkatalysators eingesetzte Trägerkörper während des Aufsprühens erwärmt, beispielsweise mittels erwärmtem Prozessgas. Das Prozessgas weist hier vorzugsweise eine Temperatur von 30 bis 120°C, stärker bevorzugt 40 bis 110°C und am stärksten bevorzugt 50 bis 100°C auf. Die genannten Obergrenzen sollten eingehalten werden, um zu gewährleisten, dass die genannte äußere Schale eine geringe Schichtdicke aufweist.

Als Prozessgas wird vorzugsweise Luft verwendet, es können aber auch inerte Gase wie zum Beispiel Stickstoff, CO₂, Helium, Neon, Argon und deren Mischungen verwendet werden.

Das Aufsprühen der Washcoat-Suspension wird in dem erfindungsgemäßen Verfahren vorzugsweise durch Zerstäuben der Suspension mit Hilfe einer Sprühdüse bewerkstelligt. Hierbei wird vorzugsweise eine Ringspaltdüse verwendet, die eine Sprühwolke versprüht, deren Symmetrieebene vorzugsweise parallel zur Ebene des Gerätebodens verläuft. Durch den 360°-Umfang der Sprühwolke können die mittig herabfallenden Trägerkörper besonders gleichmäßig mit der Lösung besprüht werden. Dabei ist die Ringspaltdüse, d.h. deren Mündung, vorzugsweise vollständig in der die Umwälzbewegung der Trägerkörper durchführenden Apparatur eingebettet.

Durch den Schritt (c) der Katalysatorherstellung wird der gecoatete Katalysator aktiviert, besonders bevorzugt bei etwa 610°C für etwa 3 Stunden in Luft. Danach kann der Katalysator eingesetzt werden.

Die vorliegende Erfindung betrifft auch einen Schalenkatalysator, der einen Träger und eine katalytisch-aktive Schale darauf umfasst, worin die katalytisch-aktive Schale ein Bismut-Molybdän-Nickel-Mischoxid oder ein Bismut-Molybdän-Cobalt-Mischoxid, und SiO₂ enthält, wobei das SiO₂ ein Porenvolumen im Bereich von 0,1 bis 10 ml/g und eine durchschnittliche Teilchengröße im Bereich 3 bis 20 µm aufweist. Die in Verbindung mit dem Verfahren zur Herstellung des Kompositmaterials angegebenen bevorzugten Parameter des SiO₂, das darin in Schritt (d) eingesetzt wird, sind auch hier bevorzugt. Der erfindungsgemäße Schalenkatalysator ist vorzugsweise einer, der durch das erfindungsgemäße Verfahren zur Herstellung eines Schalenkatalysators hergestellt wurde bzw. dadurch erhältlich ist.

Es ist bevorzugt, dass der Anteil der katalytisch-aktiven Schale im Bereich von 15 bis 45 Gew.-%, stärker bevorzugt 20 bis 40 Gew.-% bei dem erfindungsgemäßen Schalenkatalysator liegt, bezogen auf das Gesamtgewicht des Schalenkatalysators.

Die Dicke der Schale des erfindungsgemäßen Schalenkatalysators liegt vorzugsweise im Bereich von 100 bis 1500 pm, stärker bevorzugt 300 bis 1000 µm und noch stärker bevorzugt 500 bis 900 µm. Weiterhin ist es bevorzugt, dass die Porosität der katalytisch-aktiven Schale des erfindungsgemäßen Schalenkatalysators größer als 40 %, stärker bevorzugt größer als 50 % ist. Weiterhin beträgt das Porenvolumen der katalytisch-aktiven Schale vorzugsweise 0,45 bis 0,75 m³/g, stärker bevorzugt 0,5 bis 0,7 m³/g und am stärksten bevorzugt 0,55 bis 0,67 m³/g. Der durchschnittliche Porenradius in der Schale des Schalenkatalysators liegt im Bereich von 150 bis 210 nm, stärker bevorzugt 160 bis 200 nm und am stärksten bevorzugt 170 bis 190 nm. Die Bestimmungen erfolgten hierbei gemäß DIN 66133 (Hg-Porosimetrie). Die BET-Oberfläche des erfindungsgemäßen Schalenkatalysators liegt vorzugsweise im Bereich von 10 bis 60 m²/g, stärker bevorzugt 20 bis 50 m²/g und am stärksten bevorzugt im Bereich von 20 bis 40 m²/g. Die BET-Oberfläche wird nach der 1-Punkt-Methode durch Absorption von Stickstoff nach DIN 66132 bestimmt.

Eine weitere Ausführungsform der vorliegenden Erfindung betrifft auch die Verwendung des erfindungsgemäßen Schalenkatalysators bzw. eines nach den Schritten des erfindungsgemäßen Verfahrens zur Herstellung eines Schalenkatalysators hergestellten Schalenkatalysators zur Herstellung von α,β-ungesättigten Aldehyden aus Olefinen, insbesondere die Herstellung von Acrolein aus Propen.

In anderen Worten betrifft die vorliegende Erfindung also auch ein Verfahren zur Herstellung von α,β-ungesättigten Aldehyden aus Olefinen, bzw. von Acrolein aus Propen unter Verwendung eines erfindungsgemäßen Schalenkatalysators.

Die vorstehende Erfindung soll nun anhand von Figuren und Beispielen näher erläutert werden, die jedoch nicht einschränkend für den Schutzumfang der Ansprüche ausgelegt werden sollen.

Figuren:
- Fig. 1:: Fig. 1 zeigt in einem Diagramm die Umsetzung von Propen in Abhängigkeit von der Reaktortemperatur unter Verwendung eines erfindungsgemäßen Schalenkatalysators nach Beispiel 3 und unter Verwendung eines Vollextrudatkatalysators nach Vergleichsbeispiel 1.
- Fig. 2:: Fig. 2 zeigt die Summe der Selektivität zu Acrolein und Acrylsäure in Abhängigkeit von der Menge an umgesetztem Propen eines erfindungsgemäßen Schalenkatalysators nach Beispiel 3 und eines Vollextrudatkatalysators nach Vergleichsbeispiel 1.
- Fig. 3:: Fig. 3 zeigt die Summe der Ausbeute an Acrolein und Acrylsäure in Abhängigkeit von der Menge an verbrauchtem Propen für einen erfindungsgemäßen Schalenkatalysator nach Beispiel 3 und einen Vollextrudatkatalysator nach Vergleichsbeispiel 1.
- Fig. 4:: Fig. 4 zeigt in einem Diagramm die Umsetzung von Propen in Abhängigkeit von der Reaktorverweilzeit unter Verwendung eines erfindungsgemäßen Schalenkatalysators nach Beispiel 3 und unter Verwendung eines Schalenkatalysators nach Vergleichsbeispiel 2.
- Fig. 5:: Fig. 5 zeigt die Summe der Ausbeute an Acrolein und Acrylsäure in Abhängigkeit von der Menge an verbrauchtem Propen für einen erfindungsgemäßen Schalenkatalysator nach Beispiel 3 und einen Schalenkatalysator nach Vergleichsbeispiel 2.
- Fig. 6:: Fig. 6 zeigt die Summe der Selektivität zu Acrolein und Acrylsäure in Abhängigkeit von der Menge an umgesetztem Propen eines erfindungsgemäßen Schalenkatalysators nach Beispiel 3 und eines Schalenkatalysators nach Vergleichsbeispiel 2.
- Fig. 7:: Fig. 7 zeigt in einem Diagramm die Umsetzung von Propen in Abhängigkeit von der Reaktorverweilzeit unter Verwendung eines erfindungsgemäßen Schalenkatalysators nach Beispiel 3 und unter Verwendung eines Schalenkatalysators nach Vergleichsbeispiel 3.
- Fig. 8:: Fig. 8 zeigt die Summe der Selektivität zu Acrolein und Acrylsäure in Abhängigkeit von der Menge an umgesetztem Propen eines erfindungsgemäßen Schalenkatalysators nach Beispiel 3 und eines Katalysators nach Vergleichsbeispiel 3.

- Fig. 9:: Fig. 3 zeigt die Summe der Ausbeute an Acrolein und Acrylsäure in Abhängigkeit von der Menge an verbrauchtem Propen für einen erfindungsgemäßen Schalenkatalysator nach Beispiel 3 und eines Katalysators nach Vergleichsbeispiel 3.

### Beispiele:

### Beispiel 1: Herstellung eines erfindungsgemäßen Kompositmaterials:

In einem 5 Liter Becherglas werden 0,727 kg destilliertes Wasser vorgelegt und auf 60°C erhitzt. Anschließend wird 1,02 kg Eisennitrat-Nonahydrat (Honeywell; Lot: B1960) hinzugegeben. Ohne weiteres Erwärmen werden anschließend 2,37 kg Nickelnitrat-Hexahydrat (ALFA Aesar; Lot: 61101000) und 0,62 kg Magnesiumnitrat-Hexahydrat (Honeywell; Lot: 90140) hinzugegeben und gerührt, bis sich dieses ebenfalls löst. Nach erneutem Erhitzen auf 50°C werden 0,039 kg 1 M KOH (Merck; Lot: HC111978) hinzugegeben. Nach der Zugabe des Kaliumhydroxids ist ein brauner Niederschlag zu sehen, der sich aber schnell wieder auflöst. Anschließend wird 0,3 kg Bismutnitrat-Pentahydrat (ALFA Aesar; Lot: 42060004) hinzugegeben. Die Lösung wird etwa 12 Stunden bei 35°C gerührt.

In einem weiteren Ansatz werden 8,3 kg destilliertes Wasser vorgelegt. Anschließend werden 2,62 kg Ammoniumheptamolybdat-Tetrahydrat (HC Starck; Lot: 1163/048) zu der Lösung hinzugegeben und bei 35°C unter Rühren gelöst. Dazu gibt man 1,1 kg Bindzil 2034Di (Akzo Nobel; Lot: N00210). Die Messung des pH-Werts bei einer Temperatur von 33°C ergab einen pH-Wert von 5,33.

Die zuerst hergestellte über Nacht gerührte Lösung wurde mit Hilfe einer Schlauchpumpe (WATSON MARLOW 323E/D) in die frisch hergestellte Lösung mit dem Ammoniumheptamolybdat gepumpt. Die Dauer des Pumpvorgangs betrug ca. 26 Minuten. Anschließend wurden zu der erhaltenen Suspension 1,88 kg einer 0,1 % Lösung Praestol 611BC (Ashland 004041281623233) und 0,19 kg Syloid C809 (Grace Davison; Lot: 1000214955) hinzugegeben. Der pH-Wert der so erhaltenen Suspension betrug 1,26. Die so erhaltene Lösung wurde 3 Stunden gerührt bevor sie in einen Pulsationsreaktor der Firma IBU-tec (Typ: PR-4) eingesprüht wurde. Die Temperatur im Pulsationsreaktor betrug 500°C und die Verweilzeit lag im Bereich von 200 ms bis 2 s.

Die Ausbeute des so erhaltenen erfindungsgemäßen Kompositmaterials betrug nach dem Ausbringen aus dem Pulsationsreaktor 2,20 kg. Die BET-Oberfläche des Komposimaterials betrug 40 m²/g. Das Porenvolumen des Kompositmaterials betrug 0,16 cm³/g. Die Korngrößenverteilung war wie folgt:
- d₁₀ =: 122 µm
- d₅₀ =: 47 µm
- d₉₀ =: 4,2 µm

### Beispiel 2: Herstellung einer erfindungsgemäßen Washcoat-Suspension:

Im ersten Schritt der Herstellung gibt man 2,20 g Geniosil GF95 (Wacker GD18168) und 1,69 g 1 M Kaliumhydroxidlösung in 200 ml dest. H₂O.

Dann werden zunächst 128,21 g des im Beispiel 1 hergestellten Kompositmaterials, 9,62 g Coconit 300 (Mahlwerk Neubauer-Friedrich Geffers) und 3,21 g Syloid C809 (Grace Davison; Lot: 1000214955) in das im 1. Schritt hergestellte dest. Wasser/Geniosil/KOH Gemisch suspendiert.

Anschließend erfolgt eine Ultra-Turrax-Behandlung bei 8000 U/min für etwa 2,5 Minuten (T50 von IKA).).

Nach der Turrax-Behandlung wird die Suspension mit weiteren 300 ml dest. H₂O in ein Becherglas umgefüllt und 28,28 g Bindzil 2034 Di (N00210) zugegeben. Anschließend wird kurz gerührt und dann 25,64 g des organischen Binders EP 16 (Fa. Wacker) zugegeben.

### Beispiel 3: Herstellung eines erfindungsgemäßen Schalenkatalysators:

Es werden 200 g 4,5 mm großer Steatitsphären (EXACER #N.27/11) in einem Aircoater 025 der Firma Innojet mit Luft zu einem Fließbett verwirbelt, wobei die Temperatur der Prozessluft 90°C betrug. Anschließend wurde bei einem Düsendruck von 1,0 bar bei einer Sprührate von 4 g/min pro 200 g zu beschichtendem Trägerkörper die nach Beispiel 2 hergestellte Washcoat-Suspension eingesprüht.

Nach dem Ende des Sprühvorgangs wird die Verwirbelung durch die Prozessluft gestoppt, die beschichteten Trägerkörper aus der Beschichtungsvorrichtung ausgebracht und anschließend in einem Kalzinierofen zunächst 2 Stunden bei 400°C und dann anschließend 3 Stunden bei 610°C kalziniert.

Die Schalendicke der katalytisch-aktiven Beschichtung betrug 761 µm und der Gewichtsanteil der aufgebrachten Beschichtung betrug 27,4 %.

Die Bestimmung der Schalendicke erfolgt in der vorliegenden Anmeldung mittels der sogenannten Schiebelehre. Hierbei wird mit einer elektronischen Schiebelehre der Durchmesser von 40 Katalysatorkugeln gemessen, gemittelt und anschließend der Durchmesser der reinen Trägerkugeln subtrahiert.

### Vergleichsbeispiel 1: Herstellung eines Vollextrudatkatalysators:

Zunächst wurde gemäß dem Beispiel 2 der DE 10 2008 017 308 ein Bismut-Molybdän-Mischoxid-Pulver hergestellt.

400 g des Bismut-Molybdän-Mischoxid-Pulver wird zusammen mit 23,53 gg Syloid C809 (Grace Davison 1000214955), 35,29 g Zusoplast C 92 (ZSCHWIMMER & SCHWARZ Ch. 143048 001) und 11,76 g Maisstärke (Charge QF05012412) in einen Kneter (Herrman Linde Kneter Typ: LKII2) gegeben und für 5 Minuten trocken durchmischt. Anschließend werden 117,65 g Ludox AS 40 (Grace Davison Lot: 510311) zugegeben. Dann wird so lange Wasser zugegeben bis eine extrudierfähige Masse entsteht (250 ml, ca. alle 3 min 50 ml). Nach der Zugabe von 200 ml Wasser wird 15 min geknetet und nochmals 50 ml in Portionen zugegeben Anschließend wird 14, 12 g g Steatitöl (Freidling von PA 10.12.10) dazugegeben und weitere 5 Minuten geknetet. (gesamte Knetzeit: 40 min).

Die so erhaltene Knetmasse wird nach und nach in einen ECT Extruder gegeben. Matrize: 6 mm Extrudate, 3 Loch Matrize; Einstellungen: Antrieb Schnecke: 30 U/min; Druck 7 bis 8 bar.

Die erhaltenen Extrudate werden anschließend in einem VENTI-Line Trockenschrank einen Tag bei 120°C getrocknet. Anschließend werden die getrockneten Extrudate in einem Nabertherm Umluftofen 8 h bei 590°C kalziniert. Die Kalzinierung führt zu einem Gewichtsverlust der Extrudate von ca. 11%.

### Vergleichsbeispiel 2: Herstellung eines nichterfindungsgemäßen Schalenkatalysators:

Zunächst wurde gemäß dem Beispiel 1 ein Kompositmaterial hergestellt, mit dem Unterschied, dass dazu hierzu keine Porenbildner eingesetzt wurden und die Kalzinierung nicht in einem Pulsationsreaktor der Firma IBU-tec, sondern in einem Kalzinierofen durchgeführt wurde.

Gemäß dem Beispiel 2 wurde aus dem so hergestellten Kompositmaterial eine Washcoatsupension hergestellt, mit dem Unterschied, dass auch hier keine Porenbildner hinzugegeben wurden.

Analog dem Beispiel 3 wurde mit der so erhaltenen Washcoatsuspension ein Schalenkatalysator hergestellt.

### Vergleichsbeispiel 3:

Zunächst wurde gemäß dem Beispiel 1 ein Kompositmaterial hergestellt, mit dem Unterschied, dass dazu hierzu keine Porenbildner eingesetzt wurden. Gemäß dem Beispiel 2 wurde aus dem so hergestellten Kompositmaterial eine Washcoatsupension hergestellt, mit dem Unterschied, dass auch hier keine Porenbildner hinzugegeben wurden.

Analog dem Beispiel 3 wurde mit der so erhaltenen Washcoatsuspension ein Schalenkatalysator hergestellt.

### Katalytische Tests:

Der erfindungsgemäße Schalenkatalysator nach Beispiel 3 und der Vollextrudatkatalysator nach den Vergleichsbeispielen 1 bis 3 wurden hinsichtlich ihrer katalytischen Performance bei der Umsetzung von Propen zu Acrolein bzw. Acrylsäure untersucht. Hierbei betrug die Zuführzusammensetzung 8,0 Vol.-% Wasser, 9,0 % Propen und 14,4 % Sauerstoff, wobei der Rest Inertgas war. Die Gesamtflussrate betrug hierbei 78 ml/min pro jedem Rohr der Katalysatorvorrichtung.

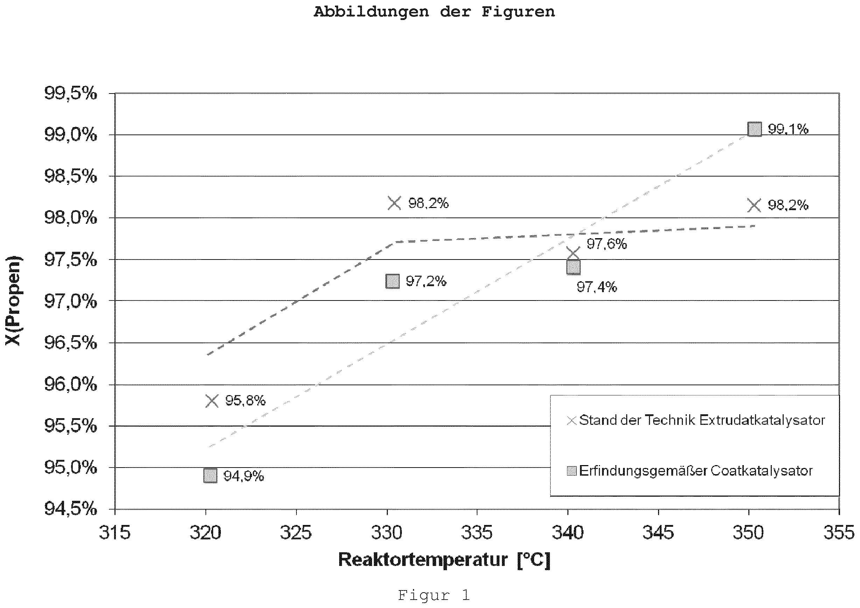

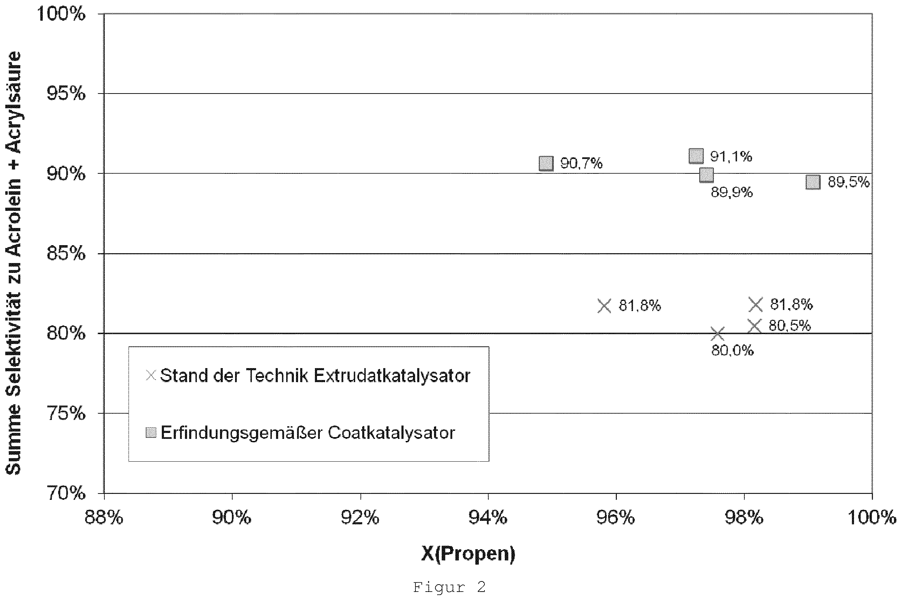

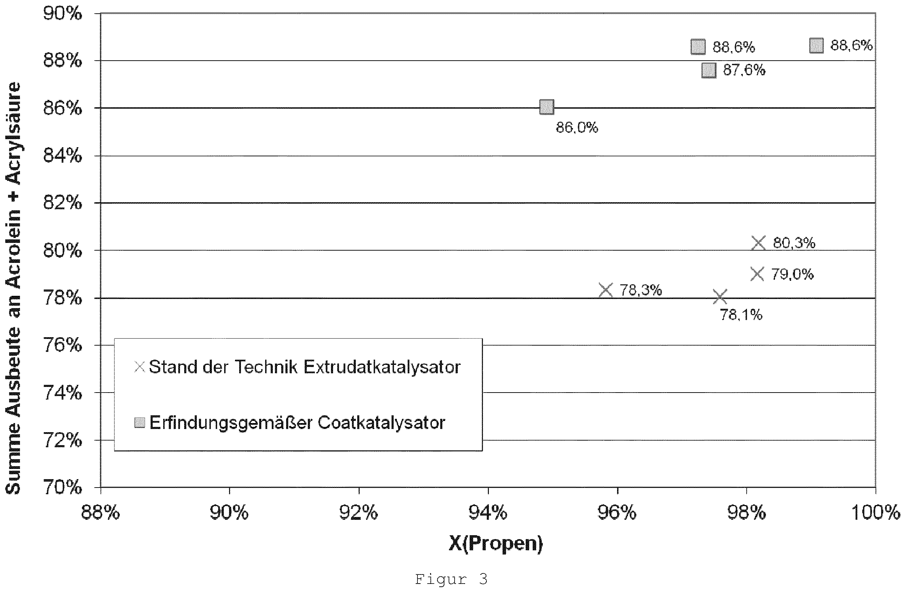

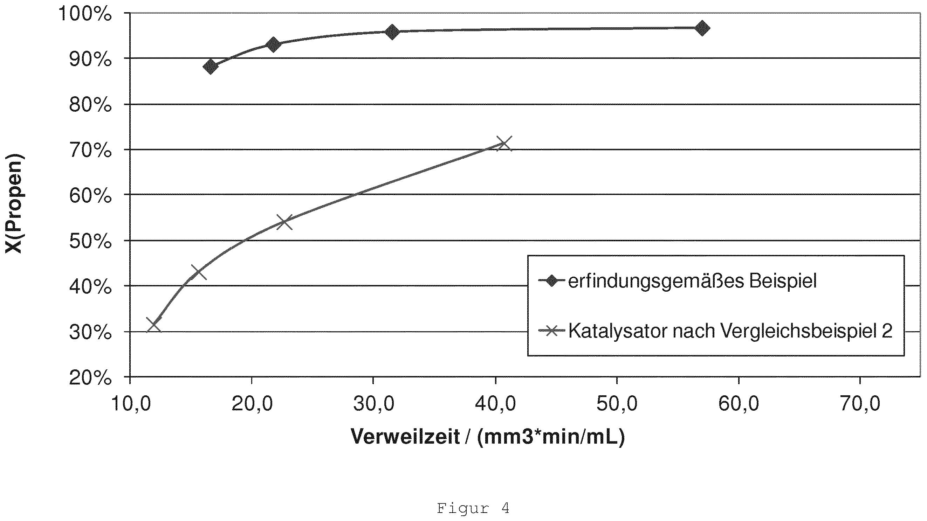

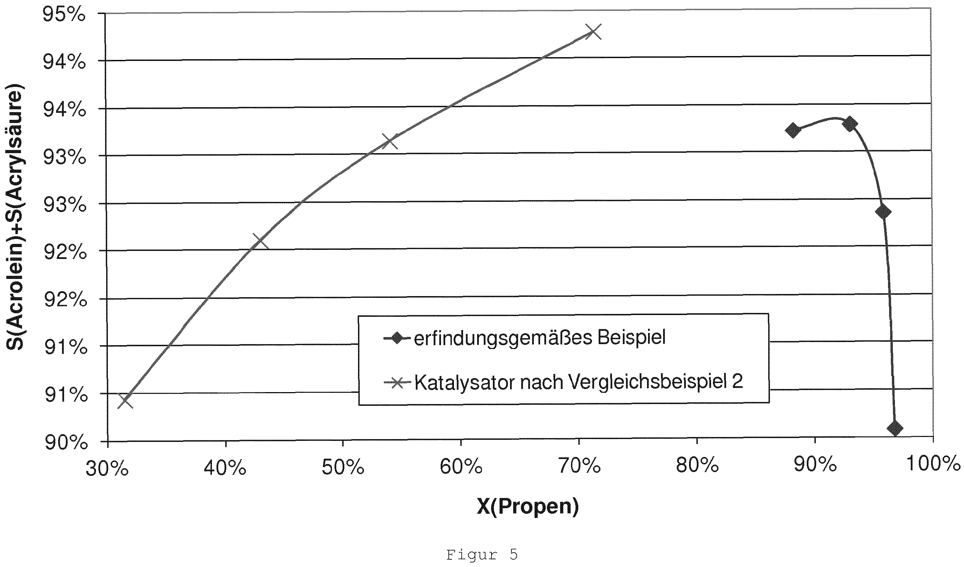

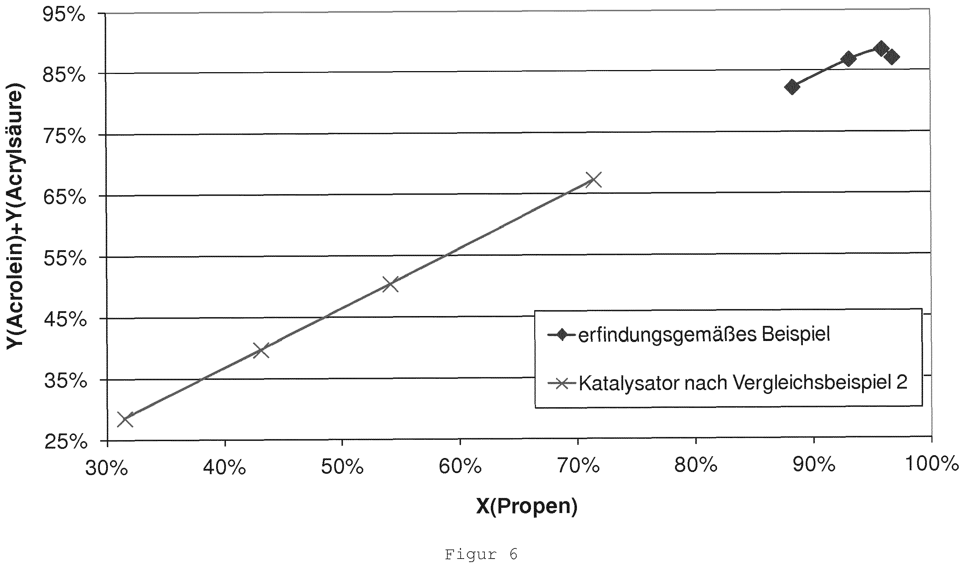

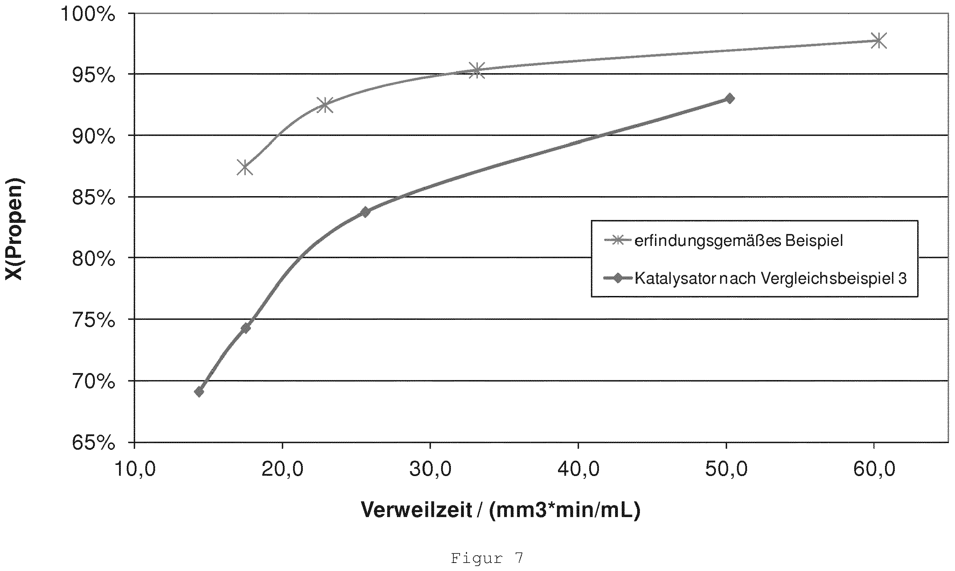

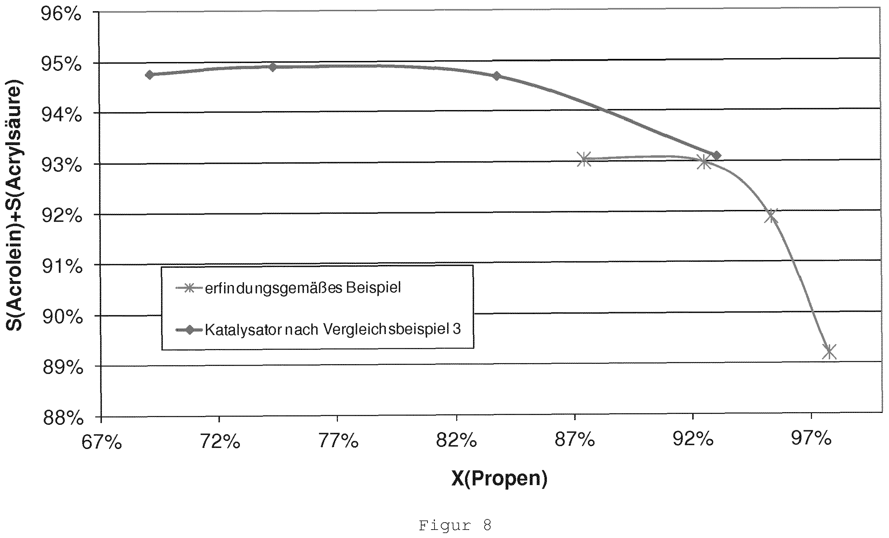

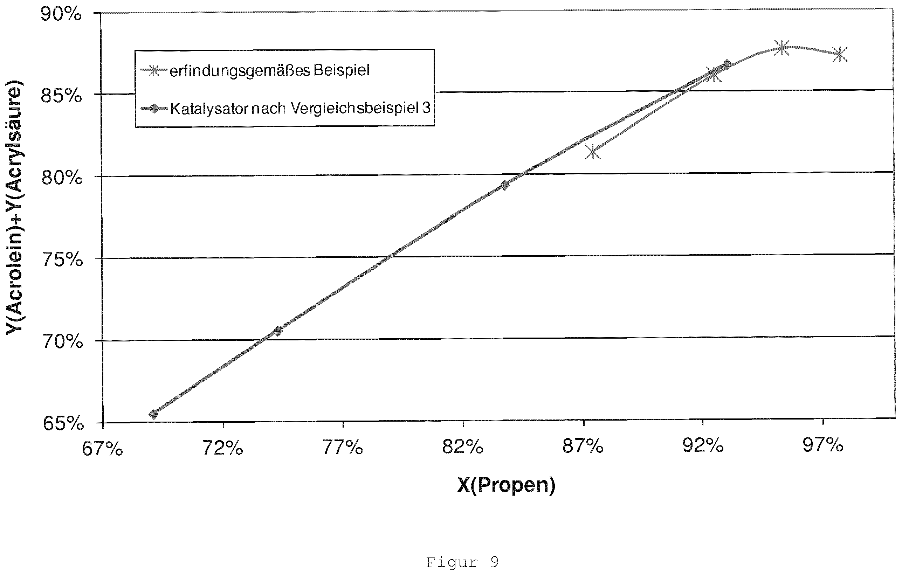

Die katalytische Performance des erfindungsgemäßen Schalenkatalysators und des Vollextrudatkatalysators sind in den Fig. 1 bis 3 zu sehen. Die katalytische Performance des erfindungsgemäßen Schalenkatalysators und des Katalysators nach Vergleichsbeispiel 2 sind in den Fig. 4 bis 6 zu sehen. Die katalytische Performance des erfindungsgemäßen Schalenkatalysators und des Katalysators nach Vergleichsbeispiel 3 sind in den Fig. 7 bis 9 zu sehen.

## Patentansprüche

1. Verfahren zur Herstellung eines Kompositmaterials umfassend die folgenden Schritte:
(a) Herstellen einer ersten wässrigen Lösung enthaltend Salze des Bismuts und Nickels oder des Bismuts und Cobalts sowie zusätzlich ein Salz des Magnesiums;
(b) Herstellen einer zweiten wässrigen Lösung enthaltend eine Molybdänverbindung und gegebenenfalls ein Bindemittel;
(c) Zugabe der ersten wässrigen Lösung zu der zweiten wässrigen Lösung, wobei eine erste Suspension gebildet wird;
(d) Zugabe einer zweiten Suspension zu der ersten Suspension unter Bildung einer dritten Suspension, wobei die zweite Suspension SiO₂ enthält, das ein Porenvolumen im Bereich von 0,1 bis 10 ml/g und eine durchschnittliche Teilchengröße im Bereich von 3 bis 20 µm aufweist; und
(e) Sprühkalzinieren der dritten Suspension bei einer Temperatur im Bereich von 300 bis 600°C unter Erhalt des Kompositmaterials enthaltend ein Bismut-Molybdän-Nickel-Mischoxid oder Bismut-Molybdän-Cobalt-Mischoxid.

2. Verfahren nach Anspruch 1, worin in Schritt (a) die erste wässrige Lösung zusätzlich ein Salz des Eisens enthält.

3. Verfahren nach Anspruch 1 oder 2, worin in Schritt (a) die erste wässrige Lösung eine Kaliumverbindung enthält.

4. Verfahren nach einem der Ansprüche 1 bis 3, worin in Schritt (b) die zweite wässrige Lösung zusätzlich ein Bindemittel enthält.

5. Verfahren nach einem der Ansprüche 1 bis 4, worin zusätzlich zu der zweiten Suspension in Schritt (d) auch ein Fällungsmittel zu der ersten Suspension hinzugegeben wird.

6. Kompositmaterial erhältlich nach einem Verfahren der Ansprüche 1 bis 5.

7. Verwendung des Kompositmaterials nach Anspruch 6 zur Herstellung einer Washcoat-Suspension.

8. Washcoat-Suspension enthaltend ein Kompositmaterial nach Anspruch 6 und entionisiertes Wasser.

9. Verfahren zur Herstellung eines Schalenkatalysators umfassend die folgenden Schritte:
(a) Herstellen einer Washcoat-Suspension nach Anspruch 8;
(b) Beschichten von Trägerkörpern mit der in Schritt (a) hergestellten Washcoat-Suspension; und
(c) Kalzinieren der beschichteten Trägerkörper bei einer Temperatur im Bereich von 500 bis 700°C.

10. Verfahren nach Anspruch 9, worin beim Herstellen der Washcoat-Suspension in Schritt (a) zusätzlich ein Bindemittel hinzugegeben wird.

11. Verfahren nach Anspruch 9 oder 10, worin das Beschichten des Trägerkörpers durchgeführt wird, indem auf eine einer Umwälzbewegung unterzogenen Schüttung von Trägerkörpern die in Schritt (a) hergestellte Washcoatsuspension aufgesprüht wird.

12. Schalenkatalysator umfassend einen Trägerkörper und eine katalytisch-aktive Schale darauf, worin die katalytisch-aktive Schale das Kompositmaterial gemäß Anspruch 6 umfasst.

13. Schalenkatalysator nach Anspruch 12, worin die Porosität der katalytisch-aktiven Schale größer als 60% ist.

14. Schalenkatalysator nach Anspruch 12 oder 13, worin der Anteil der katalytisch-aktiven Schale im Bereich von 15 bis 45 Gew.-% liegt, bezogen auf das Gesamtgewicht des Schalenkatalysators.

15. Schalenkatalysator nach einem der Ansprüche 12 bis 14, worin die Dicke der Schale im Bereich von 100 bis 1500 µm liegt.

16. Verwendung eines Schalenkatalysators nach einem der Ansprüche 12 bis 15 zur Herstellung von α,β-ungesättigten Aldehyden aus Olefinen.

## Claims

1. Process for producing a composite material, comprising the following steps:
(a) preparing a first aqueous solution comprising salts of bismuth and of nickel or of bismuth and of cobalt and further comprising a salt of magnesium;
(b) preparing a second aqueous solution comprising a molybdenum compound and optionally a binder;
(c) adding the first aqueous solution to the second aqueous solution, forming a first suspension;
(d) adding a second suspension to the first suspension to form a third suspension, the second suspension comprising SiO₂ which has a pore volume in the range from 0.1 to 10 ml/g and an average particle size in the range from 3 to 20 µm; and
(e) spray-calcining the third suspension at a temperature in the range from 300 to 600°C, to give the composite material comprising a bismuth-molybdenum-nickel mixed oxide or bismuth-molybdenum-cobalt mixed oxide.

2. Process according to Claim 1, wherein in step(a) the first aqueous solution further comprises a salt of iron.

3. Process according to Claim 1 or 2, wherein in step (a) the first aqueous solution comprises a potassium compound.

4. Process according to any of Claims 1 to 3, wherein in step (b) the second aqueous solution further comprises a binder.

5. Process according to any of Claims 1 to 4, wherein, in addition to the second suspension, in step (d), a precipitant is also added to the first suspension.

6. Composite material obtainable by a process of Claims 1 to 5.

7. Use of the composite material according to Claim 6 for producing a washcoat suspension.

8. Washcoat suspension comprising a composite material according to Claim 6 and deionized water.

9. Process for producing a coated catalyst, comprising the following steps:
(a) producing a washcoat suspension according to Claim 8;
(b) coating support bodies with the washcoat suspension produced in step (a); and
(c) calcining the coated support bodies at a temperature in the range from 500 to 700°C.

10. Process according to Claim 9, wherein, during the production of the washcoat suspension in step (a), a binder is additionally added.

11. Process according to Claim 9 or 10, wherein the coating of the support body is carried out by spraying the washcoat suspension produced in step (a) onto a bed of support bodies that is subject to a circulatory motion.

12. Coated catalyst comprising a support body and a catalytically active shell thereon, wherein the catalytically active shell comprises the composite material according to Claim 6.

13. Coated catalyst according to Claim 12, wherein the porosity of the catalytically active shell is greater than 60%.

14. Coated catalyst according to Claim 12 or 13, wherein the fraction of the catalytically active shell is in the range from 15 to 45 wt%, based on the total weight of the coated catalyst.

15. Coated catalyst according to any of Claims 12 to 14, wherein the thickness of the shell is in the range from 100 to 1500 µm.

16. Use of a coated catalyst according to any of Claims 12 to 15 for preparing α,β-unsaturated aldehydes from olefins.

## Revendications

1. Procédé de fabrication d'un matériau composite, comprenant les étapes suivantes :
(a) la fabrication d'une première solution aqueuse contenant des sels de bismuth et de nickel ou de bismuth et de cobalt, et en outre un sel de magnésium ;
(b) la fabrication d'une deuxième solution aqueuse contenant un composé de molybdène et éventuellement un liant ;
(c) l'ajout de la première solution aqueuse à la deuxième solution aqueuse, une première suspension étant formée ;
(d) l'ajout d'une deuxième suspension à la première suspension pour former une troisième suspension, la deuxième suspension contenant du SiO₂, qui présente un volume de pores dans la plage allant de 0,1 à 10 ml/g et une taille de particule moyenne dans la plage allant de 3 à 20 µm ; et
(e) la calcination par pulvérisation de la troisième suspension à une température dans la plage allant de 300 à 600 °C pour obtenir le matériau composite contenant un oxyde mixte de bismuth-molybdène-nickel ou un oxyde mixte de bismuth-molybdène-cobalt.

2. Procédé selon la revendication 1, dans lequel, à l'étape (a), la première solution aqueuse contient en outre un sel de fer.

3. Procédé selon la revendication 1 ou 2, dans lequel, à l'étape (a), la première solution aqueuse contient un composé de potassium.

4. Procédé selon l'une quelconque des revendications 1 à 3, dans lequel, à l'étape (b), la deuxième solution aqueuse contient en outre un liant.

5. Procédé selon l'une quelconque des revendications 1 à 4, dans lequel un agent de précipitation est ajouté à la première suspension à l'étape (d) en plus de la deuxième suspension.

6. Matériau composite pouvant être obtenu par un procédé selon les revendications 1 à 5.

7. Utilisation du matériau composite selon la revendication 6 pour la fabrication d'une suspension d'imprégnation.

8. Suspension d'imprégnation contenant un matériau composite selon la revendication 6 et de l'eau déionisée.

9. Procédé de fabrication d'un catalyseur à enveloppe comprenant les étapes suivantes :
(a) la fabrication d'une suspension d'imprégnation selon la revendication 8 ;
(b) le revêtement de corps supports avec la suspension d'imprégnation fabriquée à l'étape (a) ; et
(c) la calcination des corps supports revêtus à une température dans la plage allant de 500 à 700 °C.

10. Procédé selon la revendication 9, dans lequel un liant est en outre ajouté lors de la fabrication de la suspension d'imprégnation à l'étape (a).

11. Procédé selon la revendication 9 ou 10, dans lequel le revêtement du corps support est réalisé par pulvérisation de la suspension d'imprégnation fabriquée à l'étape (a) sur un chargement de corps supports soumis à un mouvement de rotation.

12. Catalyseur à enveloppe comprenant un corps support et une enveloppe catalytiquement active sur celui-ci, dans lequel l'enveloppe catalytiquement active comprend le matériau composite selon la revendication 6.

13. Catalyseur à enveloppe selon la revendication 12, dans lequel la porosité de l'enveloppe catalytiquement active est supérieure à 60 %.

14. Catalyseur à enveloppe selon la revendication 12 ou 13, dans lequel la proportion de l'enveloppe catalytiquement active se situe dans la plage allant de 15 à 45 % en poids, par rapport au poids total du catalyseur à enveloppe.

15. Catalyseur à enveloppe selon l'une quelconque des revendications 12 à 14, dans lequel l'épaisseur de l'enveloppe se situe dans la plage allant de 100 à 1 500 µm.

16. Utilisation d'un catalyseur à enveloppe selon l'une quelconque des revendications 12 à 15 pour la fabrication d'aldéhydes α,β-insaturées à partir d'oléfines.
